# EUROPEAN PATENT APPLICATION

(11) **EP 1 607 075 A1**
(43) Date of publication of application: **21.12.2005**
(21) Application number: 05104282.8
(22) Date of filing: 19.05.2005
(51) Int. Cl.: A61F 9/007

(54) **Handpiece tip**

(30) Priority: 14.06.2004 US 867291
(71) Applicant: Alcon Inc., 6331 Hunenberg (CH)
(72) Inventor: Ghannoum, Ziad R., CA 92679, Trabuco Canyon (US); Schneider, Marina L., PA 19341, Exton (US)
(74) Representative: Hanna, Peter William Derek

(57) **Abstract**

A handpiece tip (10) is provided having a seal (15), a raised ridge or snap lock (16) and a stop (20). Such a construction allows an internally threaded infusion sleeve (12) to be pushed onto the handpiece tip without threading. The raised ridge locks the infusion sleeve into place. The seal prevents leakage from the connection. The stop prevents the infusion sleeve from being pushed too far onto the handpiece tip. The handpiece tip can be used with conventional internally threaded infusion sleeves.

## Description

### Background of the Invention

This invention relates generally to the field of cataract and lens removal surgery and more particularly to handpiece tips and infusion sleeves.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubes. Through the electric cable, the console varies the power level transmitted by the handpiece to the attached cutting tip and the flexible tubes supply irrigation fluid to, and draw aspiration fluid from, the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The distal tip of the nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the handpiece tip. Other non-ultrasonic handpieces, such as laser phaco and liquefracture, use a similar construction.

The threaded connection between the handpiece tip and the infusion sleeve used in the prior art works extremely well, but is unnecessarily difficult and time consuming to manipulate. Therefore, a need continues to exist for a simpler connection between the handpiece tip and the infusion sleeve.

### Brief Summary of the Invention

The present invention improves upon the prior art by providing a handpiece tip having a seal, a raised ridge or snap lock and a stop, in accordance with claims which follow. Such a construction allows the infusion sleeve to be pushed onto the handpiece tip without threading. The raised ridge locks the infusion sleeve into place. The seal prevents leakage from the connection. The stop prevents the infusion sleeve from being pushed too far onto the handpiece tip. The handpiece tip can be used with conventional internally threaded infusion sleeves.

Accordingly, one objective of the present invention is to provide a handpiece tip for a surgical handpiece having a snap lock feature.

Another objective of the present invention is to provide a handpiece tip for a surgical handpiece having a stop.

Another objective of the present invention is to provide a handpiece tip for a surgical handpiece having a seal.

These and other advantages and objectives of the present invention will become apparent from the detailed description and claims that follow.

### Brief Description of the Drawings

FIG. 1 is a side elevational view of the handpiece tip of the present invention receiving an infusion sleeve.
FIG. 2 is a cross-sectional view of the handpiece tip of the present invention illustrating an infusion sleeve installed on the handpiece tip.

### Detailed Description of the Invention

Handpiece tip 10 of the present invention may be used in combination with any of a variety of ultrasound and non-ultrasound handpieces, such as the handpieces used in the UNIVERSAL® , LEGACY® , INFINITI® and AQUALASE® surgical systems commercially available from Alcon Laboratories, Inc., Fort Worth, Texas.

One suitable handpiece tip is described in our co-pending European Patent Application No. EP-A-1,506,757, which describes a tip assembly for a liquefaction surgical handpiece. The tip assembly has an inner connector and an outer cap. The outer cap contains an external thread for attaching the tip assembly to a handpiece. The inner housing contains an alignment tab and fits within the outer cap so as to allow rotation of the inner connector within the outer cap. The inner and outer liquefaction tip tubes are frictional fit into a bore in the inner connector. The proximal end of the inner housing contains a gasket that seals the inner housing against the handpiece.

Handpiece tip 10 of the present invention has body 11 made from any suitable material such as plastic, stainless steel or titanium and is designed to receive infusion sleeve 12, which may be of conventional construction (e.g., silicone rubber). Body 11 may be generally hollow and having a hub 13 integrally formed with reduced diameter shaft 17.

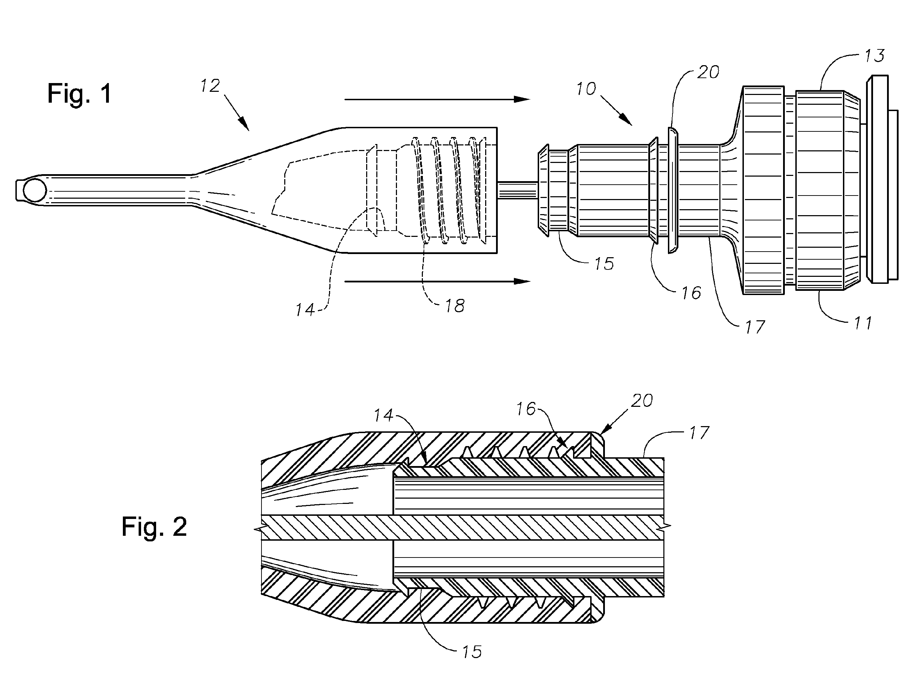

As best seen in FIG. 2, shaft 17 includes fluid seal 15 in the form of a circumferential trough or groove extending circumferentially around shaft 17. Located proximally from seal 15 is snap lock 16, which consists of a circumferential raised ridge or lip. Snap lock 16 is sized and shaped to be received in internal threads 18 of infusion sleeve 12 so as to lock infusion sleeve 12 on shaft 17 and to act as a secondary fluid seal. The primary fluid seal is formed by circumferential rib 14 in infusion sleeve 12 that snaps into place within seal 15. Raised flange 20 acts as a stop, and prevents infusion sleeve 12 from being pushed too far onto shaft 17.

Such a construction allows an internally threaded infusion sleeve 12 to be pushed onto the handpiece tip 10 without screwing or threading.

This description is given for purposes of illustration and explanation. It will be apparent to those skilled in the relevant art that changes and modifications may be made to the invention described above without departing from its scope.

## Claims

1. A tip (10) for a handpiece, comprising:
a) a body (11), the body having a hub (13) and an elongated shaft (17) projecting out from the body;
b) a snap lock (16) on the shaft; and
c) a fluid seal (15) on the shaft.

2. The handpiece tip of claim 1, wherein the snap lock (16) comprises a circumferential raised ridge or lip.

3. The handpiece of claim 2, wherein, in use, the snap lock (16) is sized and shaped to be received in internal threads (18) of an infusion sleeve (12) so as to lock infusion sleeve (12) on shaft (17) and to act as a secondary fluid seal.

4. The handpiece tip of claim 1 or claim 2, wherein the fluid seal (15) comprises a circumferential groove.

5. The handpiece tip of claim 4, wherein, in use, a primary fluid seal is formed by a circumferential rib (14) in an infusion sleeve (12) adapted to snap into place within the circumferential groove of the fluid seal (15), when the sleeve is received by the tip.

6. The handpiece tip of any one of claims 1 to 3, further comprising a stop (20) on the shaft (17).

7. The handpiece tip of claim 6, wherein the stop (20) comprises a raised flange.

8. The handpiece of claim 6 or claim 7, wherein, in use, when an infusuion sleeve (12) is received by the tip, the stop (20) is arranged to prevent the infusion sleeve (12) from being pushed too far onto the shaft (17).
